(19) Europäisches Patentamt
European Patent Office
Office européen des brevets

(11) EP 2 263 639 B1

(12) **EUROPEAN PATENT SPECIFICATION**

(45) Date of publication and mention of the grant of the patent:
**06.02.2019 Bulletin 2019/06**

(21) Application number: **10166484.5**

(22) Date of filing: **18.06.2010**

(51) Int Cl.:
***A61K 8/06*** *(2006.01)* ***A61K 8/25*** *(2006.01)*
***A61K 8/26*** *(2006.01)* ***A61K 8/81*** *(2006.01)*
***A61K 8/87*** *(2006.01)* ***A61K 8/88*** *(2006.01)*
***A61K 8/891*** *(2006.01)* ***A61Q 19/00*** *(2006.01)*

(54) **Water-in-oil emulsified cosmetic comprising hydrophobic powder**

Wasser-in-Öl emulgierte Kosmetik enthaltend ein hydrophobes Pulver

Cosmétique émulsifié eau dans l'huile comprenant une poudre hydrophobe

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **19.06.2009 JP 2009146694**

(43) Date of publication of application:
**22.12.2010 Bulletin 2010/51**

(73) Proprietor: **Kao Corporation**
**Chuo-Ku**
**Tokyo 103-8210 (JP)**

(72) Inventor: **Okubo, Koji**
**Tokyo 131-8501 (JP)**

(74) Representative: **Hoffmann Eitle**
**Patent- und Rechtsanwälte PartmbB**
**Arabellastraße 30**
**81925 München (DE)**

(56) References cited:
**WO-A1-2007/064687** **WO-A2-2010/108088**
**JP-A- 2007 023 022** **US-A1- 2007 224 141**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to a water-in-oil emulsified cosmetic.

### BACKGROUND OF THE INVENTION

Cosmetics for supplying moisture or oil to the skin are provided as a cream-form emulsion in which water, a water-soluble solvent that forms various types of moisturizing agents, and oil are combined. The effect of a cosmetic felt by a user is not felt simply due to components in the composition, and depends on various physical properties of the cosmetic emulsion. For example, there are aspects of performance such as luster, smoothness when applying, and ease of conforming to the skin of a cosmetic emulsion.

On the other hand, changing the feel during use when applying has been examined by adding various powders to cosmetics. Specifically, it is known that, by applying a spherical powder onto the skin surface, silky feel, ease of conforming, non-stickiness, or smoothness of a cosmetic is improved due to the texture of the powder itself.

Japanese Patent Application JP1995-267819A and JP2006-282632A disclose techniques for improving non-stickiness when applying onto the skin by adding a spherical powder to a water-in-oil emulsified cosmetic. Furthermore, Japanese Patent Application JP1993-262622A discloses that smoothness on the skin and texture properties are improved by adding a resin powder to a cosmetic, and Japanese Patent Application JP1996-104613A discloses that a cosmetic includes a microparticulate powder and a resin powder, the resin powder in the cosmetic improves the texture of the skin and is useful for eliminating a frictional feel due to the microparticulate powder WO2007/064687 discloses (ex. 10) a water-in-oil emulsified cosmetic comprising: (A)dimethicone, (B) DC2503, (C) silicone crosspolymer,(D)KSG-210 and (E) water. However, in all of the patent applications, there is still room for improvement in terms of feel during use when applying a cosmetic onto the skin.

### SUMMARY OF THE INVENTION

The present invention, there is provided a water-in-oil emulsified cosmetic including components (A) to (E) as defined in claim 1.

In accordance with the present invention, there is provided a cosmetic that is characterized by its texture when applying, gives a feeling of richness during application to and spreading over the skin, and imparts an unprecedented feel during use.

### BRIEF DESCRIPTION OF THE DRAWINGS

The above, advantages and features of the present invention will be more apparent from the following description taken in conjunction with the accompanying drawings, in which:

Fig. 1 is a schematic cross-sectional view showing the constitution of a viscosity-measuring device.

### DETAILED DESCRIPTION OF THE INVENTION

The invention will be now described herein with reference to illustrative embodiments. Those skilled in the art will recognize that many alternative embodiments can be accomplished using the teachings of the present invention and that the invention is not limited to the embodiments illustrated for explanatory purpose.

The present invention controls the physical properties of a water-in-oil emulsified cosmetic and provides a cosmetic that is characterized by its texture when applying, gives a feel of richness during application to and spreading over the skin, and can impart an unprecedented feel during use.

The present inventors have found that, by combining an oil that is a liquid at 25°C, an oil that is a solid at 25°C, a hydrophobic powder having an average particle size of 8 to 30 μm, a surfactant having an HLB of no greater than 5, and water, a cosmetic that is characterized by its texture when applying, gives a feel of richness during application to and spreading over the skin, and imparts an unprecedented feel during use can be obtained.

The water-in-oil emulsified cosmetic of the present invention includes components (A) to (E) below. Each component is specifically explained below.

Component (A) is now explained.

In view of the skin moisture retention the oil comprises a polydimethylsiloxaxane that is no greater than 50 cSt at 25°C or fluid isoparaffin. They may be used singly or in a combination of two or more types.

Examples of further compounds if a combination at two or more types is used, include a plant oil such as jojoba oil or olive oil; an animal oil such as liquid lanolin; a straight-chain or branched hydrocarbon oil such as fluid paraffin, light isoparaffin, squalane, or squalene; an ester oil such as a fatty acid ester or a polyhydric alcohol fatty acid ester; a silicone oil such as polydimethylcyclosiloxane, polymethylphenylsiloxane, polymethyl hydrogen siloxane, or a higher alcohol-modified polyorganosiloxane; and a fluorine oil such as a fluoropolyether or a perfluoroalkyl ether silicone.

From the viewpoint of enhancing the dispersibility of a powder or a solid fat, component (A) is contained at 10 to 50 wt% in the water-in-oil emulsified cosmetic of the present invention and more preferably, in order, 11.5 to 50 wt%, 11.5 to 40 wt%, 15 to 40 wt%, and 16 to 25 wt%.

Component (B) is now explained.

Component (B) is an oil that is a solid at 25°C. As component (B), one with a melting point of at least 40°C is preferable, one with a melting point of at least 60°C is more preferable, and one with a melting point of no greater than 100°C is preferable from the viewpoint of stability.

Compound (B) comprises a ceramide or a ceramide-like compound represented by Formula (1) below. They may be used singly or in a combination of 2 or more types.

Specific examples of component (B) when a combination of 2 or more types is used include a staight-chain fatty acid having 14 to 22 carbon atoms; cholesterol and a cholesterolester such as cholesterol, cholesteryl hydroxystearate, the cholesteryl ester of Macadamia nut oil fatty acid, di(cholesteryl/behenyl/octyldodecyl) *N*-lauroyl-L-glutamate, di(cholesteryl/behenyl/octyldodecyl) *N*-lauroyl-L-glutamate; a natural ceramide such as ceramide I to ceramide VI.

[Chem. 1]

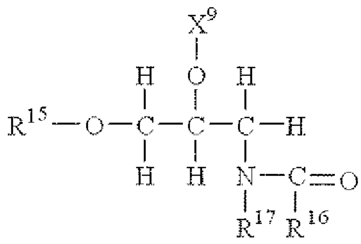

(1)

(In the formula, R15 denotes a hydrogen atom or a straight-chain, branched-chain, or cyclic saturated or unsaturated hydrocarbon group having 10 to 22 carbon atoms that may optionally be substituted with a hydroxy group; X9 denotes a hydrogen atom, an acetyl group, or a glyceryl group; R16 denotes a straight-chain, branched-chain, or cyclic saturated or unsaturated hydrocarbon group having 5 to 22 carbon atoms that may optionally be substituted with a hydroxy group or an amino group, or one in which a straight-chain or branched-chain saturated or unsaturated fatty acid having 8 to 22 carbon atoms that may optionally be substituted with a hydroxy group is ester-bonded to the ω terminal of the above hydrocarbon group; and R17 denotes a hydrogen atom or an alkyl group having a total of 1 to 30 carbon atoms that may optionally be substituted with a hydroxy group, a hydroxyalkoxy group, an alkoxy group, or an acetoxy group.)

In Formula (1), R15 is preferably nonyl, tridecyl, pentadecyl, or hexadecyl. R16 is preferably nonyl, tridecyl, pentadecyl, an undecyl group having linoleic acid ester-bonded to the ω position, a pentadecyl group having linoleic acid ester-bonded to the ω position, a pentadecyl group having 12-hydroxystearic acid ester-bonded to the ω position, or an undecyl group having a methyl branched isostearic acid amide-bonded to the ω position. R17 is preferably hydroxyethyl, 2-hydroxyethyl, or 3-methoxypropyl.

Among them, one in which R15 is a hexadecyl group, X9 is a hydrogen atom, R16 is a pentadecyl group, R17 is a hydroxyethyl group; one in which R15 is a hexadecyl group, X9 is a hydrogen atom, R16 is a nonyl group, and R17 is a hydroxyethyl group; or a pseudo type ceramide in which R15 is a hexadecyl group, X9 is a glyceryl group, R16 is a tridecyl group, and R17 is a 3-methoxypropyl group is preferable. Furthermore, *N*-(hexadecyloxyhydroxypropyl)-*N*-hydroxyethylhexadecanamide represented by Formula (2) below, in which R15 is a hexadecyl group, X9 is a hydrogen atom, R16 is a pentadecyl group, and R17 is a hydroxyethyl group, is even more preferable.

[Chem. 2]

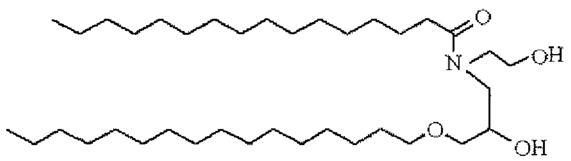

(2)

Moreover, examples of component (B) include an animal wax such as beeswax or spermaceti; a plant wax such as carnauba wax, candelilla wax, rice wax, or Japan wax; a mineral wax such as montan wax, ozokerite, ceresine, paraffin wax, petrolatum, or microcrystalline wax; and a synthetic wax such as polyethylene wax, Fischer-Tropsch wax, hardened castor oil, hydrogenated jojoba oil, stearic acid amide, or silicone wax. They may be used singly or in a combination of two or more types.

Here, it is important that component (B) is a solid fat, is in a state in which it is dispersed in component (A), and is present as a solid powder. Component (B) is obtained by dissolving component (B) in a component (A) liquid oil by heating, and subsequently cooling while stirring so as to form a precipitate.

The average particle size of component (B), from the relationship between emulsified particles and a component (C) powder, which will be described later, is preferably at least 1 μm but no greater than 30 μm, and more preferably at least 1 μm but no greater than 14 μm.

From the viewpoint of controlling the liquid properties of a cosmetic and improving stability, the content of component (B) is 0.5 to 15 wt% in the water-in-oil emulsified cosmetic of the present invention, preferably 3 to 13 wt%, and more preferably 5 to 12 wt%.

Component (C) is now explained.

Component (C) is a hydrophobic powder having an average particle size of 8 to 30μm, which comprises a urethane resin or silicone resin, and may furhter include either an inorganic powder or an organic powder.

Examples of the inorganic powder include silica, mica, talc, kaolin, sericite, titanium oxide, zinc oxide, magnesium oxide, zirconium oxide, calcium carbonate, magnesium carbonate, magnesium silicate, anhydrous silicic acid, barium sulfate, Bengala, yellow iron oxide, black iron oxide, carbon black, manganese violet, glass beads, zeolite, a pearlescent pigment (Bengala-coated mica, titanium oxide-coated mica, and so on), and a composite thereof.

Examples of the organic powder include a thermoplastic resin such as a styrene resin, an acrylic resin, a polyolefin, a fluorine resin, a polyester and a polyamide, an epoxy resin and phenolic resin. Among them, an acrylic resin, a polyamide resin, and are preferable, and for example a polymethyl methacrylate resin, polymethylsilsesquioxane, nylon, and so on can be cited. From the viewpoint of obtaining the effects of the present invention, a silicone resin and urethane resin are used.

Furthermore, from the viewpoint of improving the richness of a cosmetic, which will be described later, component (C) is preferably an elastomer powder, powder of a urethane, a silicone resin, and so on. Examples of the urethane powder include plastic powder D-400 (HDI/trimethylolhexyllactone crosspolymer) (Toshiki Pigment Co., Ltd., average particle size 13 μm) and plastic powder D-800 (HDI/trimethylolhexyllactone crosspolymer) (Toshiki Pigment Co., Ltd., average particle size 8 μm).

When the powder is hydrophilic, it may be used after subjecting it to a hydrophobizing treatment. The hydrophobizing treatment may be carried out using, for example, a hydrophobizing treatment agent such as a silicone compound (a silicone oil or a derivative thereof such as polydimethylsiloxane or polymethyl hydrogen siloxane), a fatty acid metal salt, an alkylphosphoric acid, an alkali metal salt or an amine salt of an alkylphosphoric acid, an N-mono long chain (8 to 22 carbon atom) aliphatic acyl basic amino acid, or a fluorine compound having a perfluoroalkyl group, and treatment can be carried out in accordance with a standard method.

The shape of the component (C) powder may be any of a spherical shape, a flat plate shape, or an amorphous shape, and is more preferably a spherical shape from the viewpoint of texture when applying.

From the viewpoint of improving oiliness or sticky feel during use, the average particle size of component (C) is at least 8 μm, and preferably at least 10 μm. Furthermore, the average particle size of component (C) is no greater than 30 μm, preferably no greater than 26 μm, and more preferably no greater than 15 μm.

From the viewpoint of improving oiliness or sticky feel during use, the content of component (C) is 0.5 wt% to 10 wt% in a water-in-oil emulsified cosmetic, preferably 0.5 wt% to 6.5 wt%, and more preferably 2 wt% to 5 wt%.

Component (D) is now explained.

Component (D) is a surfactant having an HLB of no greater than 5 and comprises a polyether-modified silicone. Examples of a furhter surfactant havin HLB of no greater than 5 include a glycerol fatty acid ester, a polyglycerol fatty acid ester,

a polyoxyethylene glycerol fatty acid ester, a propylene glycol fatty acid ester, polyoxyethylene polyoxypropylene glycol, a sorbitan fatty acid ester, a polyoxyethylene sorbitan fatty acid ester, polyoxyethylene castor oil, polyoxyethylene hardened castor oil, a polyoxyethylene hardened castor oil fatty acid ester, a polyethylene glycol fatty acid ester, an alkyl glyceryl ether, an alkyl polyglyceryl ether, a polyoxyethylene alkyl ether, a polyoxyethylene alkyl ether fatty acid ester, an alkylalkanolamide, a polyoxyethylene alkylamine, a fatty acid alkanolamide, and a glycerol-modified silicone.

More specific examples thereof include a polyether-modified silicone such as BY11-030 silicone (PEG/PPG-19/19 Dimethicone) (Dow Corning Toray), BY22-008M silicone (PEG/PPG-19/19 Dimethicone) (Dow Corning Toray) or SH3775M silicone (PEG-12 Dimethicone) (Dow Corning Toray), and a polyoxyethylene hardened castor oil such as Emalex HC-5 (PEG-5 hydrogenated castor oil) (Nihon Emulsion Co., Ltd.) or Emalex RWIS-315 (PEG-15 hydrogenated castor oil triisostearate) (Nihon Emulsion Co., Ltd.). Among them, BY11-030 silicone (PEG/PPG-19/19 Dimethicone) (Dow Corning Toray), SH3775M silicone (PEG-12 Dimethicone) (Dow Corning Toray) and Emalex HC-5 (PEG-5 hydrogenated castor oil) are preferable.

From the viewpoint of stably obtaining a water-in-oil emulsified cosmetic, component (D) is a surfactant having an HLB of no greater than 5.

Here, the HLB (Hydrophilic-Lipophilic Balance) shows the molecular weight of a hydrophilic group moiety relative to the total molecular weight of a surfactant and is determined from the Griffin equation.

From the viewpoint of emulsifying an aqueous phase in an oil phase and dispersing a solid fat and a powder uniformly so as to express a thickening effect, the content of component (D) in a water-in-oil emulsified cosmetic is, in order of preferance, 0.2 wt% to 10 wt%, 2 wt% to 10 wt%, 3 wt% to 10 wt%, and 3 wt% to 8 wt%. With regard to the component (D) surfactant, one type alone may be used or two or more types may be used in combination.

Component (E) is now explained. Component (E) is water, and purified water is preferably used. From the viewpoint of feel during use, the content of component (E) in a water-in-oil emulsified cosmetic is preferably at least 10 wt% but no greater than 70 wt%.

With regard to the water-in-oil emulsified cosmetic of the present invention, an aqueous phase component containing component (E) becomes emulsified particles. From the viewpoint of emulsion stability, the average emulsified particle size of the emulsified particles is at least 0.1 μm, preferably at least 0.8 μm, but no greater than 25 μm, more preferably no greater than 15 μm, and yet more preferably no greater than 13 μm.

With regard to the water-in-oil emulsified cosmetic of the present invention, among these components (A) to (D), by combining them as follows the effects of the present invention are more effectively obtained.

One containing at least polydimethylsiloxane or fluid isoparaffin as component (A), ceramide or a ceramide-like compound represented by Formula (1) as component (B), silicone resin such as a urethane resin or polymethylsilsesquioxane and so on as component (C), and a polyether-modified silicone as component (D) can be cited.

From the viewpoint of imparting an unprecedented feel during use when applying a water-in-oil emulsified cosmetic, it is important in the present invention for the proportions by weight ratio of components (A) to (E) to satisfy the relational expressions (1) to (3) below.

$$(E):((A) + (B) + (C)) = 10:90 \text{ to } 70:30 \quad (1)$$

$$0.25 \leq ((B) + (C))/(A) \leq 0.7 \quad (2)$$

$$(B) > (C) \quad (3)$$

The relationship in size between the average emulsified particle size, the average particle size of component (B), and the average particle size of component (C) is
(average particle size of component (C)) > (average particle size of component (B)) > (average emulsified particle size).

From the viewpoint of obtaining the effects of the present invention, relational expression (1) is 10:90 to 70:30, and preferably 27:73 to 70:30.

The feel during use of a cosmetic is normally obtained only after it comes into contact with and interacts with the skin in some way. In a conventional water-in-oil emulsified cosmetic, a powder is simply added to and dispersed in a cosmetic. Because of this, there is not sufficient interaction between the powder and emulsified particles of an aqueous phase. Because of this, with regard to the feel during use of a cosmetic, only the texture of the powder such as silky feel when the cosmetic is spread well is obtained. Furthermore, the viscosity of the entire water-in-oil emulsified cosmetic is sometimes enhanced by mixing a solid oil and a liquid oil and dispersing solid oil crystals in the liquid oil. However, in this case, only a thixotropic feel can be imparted at most.

The present inventor has carried out an investigation into the feel during use when a water-in-oil emulsified cosmetic is applied to the skin, and consider that by combining three types of particles, that is, the hydrophobic powder of component (C), the solid powder of component (B), and emulsified particles, interaction is caused between these particles, thereby deriving an unprecedented feel during use.

In more detail, it is surmised that, due to interaction between the hydrophobic powder of (C), the solid powder of component (B), and the emulsified particles, the structure of an emulsion is strengthened, and elasticity or a thickening action is imparted to the liquid properties of the emulsion. As a result, the strength of the emulsion structure is resilient when first applying to the skin, and it is perceived as a thickness and a strong concentrated richness.

In yet more detail, it is surmised that the effects of the present invention are obtained as follows.

In the present invention, components (B), (C), and (E) are present in a state in which they are dispersed in component (A).

Component (B) is mixed with component (A) and is melted by heating during the production process. Subsequently, by cooling while stirring, dispersion in a cosmetic as fine microparticles (amorphous) is carried out. When increasing the amount of component (B), a net-like structure is formed in the cosmetic, thus enabling the overall viscosity of the cosmetic to be increased. However, merely increasing the amount of component (B) only makes spreading when applying heavy, and richness cannot be imparted to the cosmetic.

Furthermore, when the proportion of component (E) in the cosmetic becomes high, the packing structure of emulsified particles increases. As a result, a cosmetic having highly elastic liquid properties is obtained. However, richness cannot be imparted to a cosmetic only by high elasticity.

Conventionally, when some amount of hydrophobic powder is contained in a water-in-oil emulsion, the physical properties thereof are not particularly affected. On the other hand, it is surmised that in the present invention due to interaction between component (B) and component (E), a compact structure is formed, and as a result of component (C) being dispersed in this structure, richness can be imparted to the cosmetic. In particular, it is surmised that mixing component (C), which has a larger particle size than component (B) and component (E), enables a special structure to be formed and richness to be exhibited.

The ’richness’ of the water-in-oil emulsified cosmetic of the present invention is a new texture that is sensed when it is applied and made to conform to the skin. In other words, it is a feel of a material that has never been sensed up to now and that is sensed during the process of depositing the water-in-oil emulsified cosmetic of the present invention on the skin and starting to apply it, and it improves the feel of application. It is thought that, compared with the thixotropic feel of a conventional water-in-oil emulsified cosmetic, the water-in-oil emulsified cosmetic of the present invention gives ’richness’ because the viscosity is retained to some extent from the time of starting application.

It is thought that the larger the particle size of component (C), the easier a special structure is formed, and special effects can easily be exhibited. However, when the particle size of component (C) is too large, it is recognized as roughness when it is applied, which is undesirable. It is therefore necessary to select an appropriate size. Furthermore, an elastomer powder that is loosely three-dimensionally bonded easily absorbs component (A), fluid isoparaffin, polydimethylsiloxane, etc. in particular, and is easily swollen. Because of this, the apparent particle size of component (C) can be increased, and special effects can easily be exhibited. Moreover, since such a powder is soft, there is the advantage that roughness is not sensed when it is applied.

Evaluation of the feel during use of the water-in-oil emulsified cosmetic of the present invention may be determined by, for example, measuring the storage modulus when applying shear stress. Specifically, the storage modulus G’ is measured at each strain when applying 0.1%, 10%, and 1,000% strain to a cosmetic at a frequency of 2 Hz using a 25 mm cone-plate.

Here, a strain of 10% means gentle shear conditions and denotes a state in which a cosmetic is applied to the skin and started to be spread. When the structure formed of a cosmetic is sufficiently strong, substantially the same level of storage modulus as when there is a strain of 0% is obtained. Furthermore, a strain of 1,000% means strong shear conditions and denotes a state in which a cosmetic is spread widely on the skin. In addition, it is thought that a change in storage modulus due to shear denotes retention of the previously explained structure and the result of destroying the structure.

From the viewpoint of richness when applying to the skin being sensed, the storage modulus G’ is preferably 850 to 2,000 Pa when the strain is 0.1% (conditions 1), at least 850 Pa when the strain is 10% (conditions 2), and no greater than 100 Pa when the strain is 1,000% (conditions 3). Furthermore, from the viewpoint of stronger richness being sensed, the storage modulus G’ of (conditions 2)-(conditions 3) is at least 800 Pa.

Evaluation of feel during use of the water-in-oil emulsified cosmetic of the present invention is not limited to the above-mentioned method.

From the viewpoint of ease of taking out a cosmetic when scooped up by a finger and richness being sensed, the viscosity of the water-in-oil emulsified cosmetic measured at 25°C and 5 rpm using a T-bar spindle D in the present invention is at least 500 dPa·s or in order of preferance, at least 800 dPa·s, at least 870 dPa·s, and more preferably at last 900 dPa·s. On the other hand, from such a viewpoint, it is, no greater than 3,000 dPa·s, or in order of preferance,

no greater than 2,850 dPa·s, and no greater than 2,500 dPa·s.

Moreover, the water-in-oil emulsified cosmetic of the present invention may include, in addition to the above-mentioned components (A) to (E), for example, a medically effective component, a moisturizer, a colorant, a preservative, an antioxidant, a chelating agent, a fragrance, and so on.

A method for producing the water-in-oil emulsified cosmetic of the present invention is now explained below.

Firstly, components (A), (B), (C), and (D) are mixed and heated to at least a temperature at which component (B) melts. Alternatively, after component (B) is heated and melted, components (A), (C), and (D) are mixed and dissolved while stirring until the mixture becomes uniform. This is defined as a lipophilic component. On the other hand, component (E) and a water-soluble component are mixed uniformly, and heated to the same level of temperature as that of the lipophilic component. This is defined as an aqueous component. Subsequently, the aqueous component is added to the lipophilic component while stirring, thus carrying out emulsification. Stirring at this point may, depending on the situation, be carried out using a device having high sheer such as a homogenizer. Following this, the mixture is cooled to room temperature while stirring.

In the present invention, the average particle size of component (B) may be adjusted by the time taken for cooling and the stirring force during cooling (rotational speed), and so on.

In the present invention, the average emulsified particle size of the aqueous phase component containing component (E) may be adjusted to 0.1 μm to 25 μm by the rotational speed and so on of a homogenizer and so on.

EXAMPLES

Water-in-oil emulsified cosmetics obtained in Examples and Comparative Examples, which are explained below, were subjected to the following measurements and evaluations.

(Measurement of storage modulus by means of rheometer)

FIG. 1 is a schematic cross-sectional view showing the constitution of a viscosity-measuring device. As shown in FIG. 1, strain was applied to a sample 12 by rotating a cone-plate 11 in the direction shown by the arrow in the drawing. With respect to each of the samples to which strain was applied, the storage modulus G' at that time was measured.
Evaluation method: MCR301 rheometer (Anton Parr)
Evaluation tool: CP25
cone-plate having a diameter of 25 mm
Frequency: 2 Hz
Measurement temperature: 25°C
Amount of sample: gap was adjusted to zero, and sample was adjusted so as not to overflow from the plate.
Strain: 0.1%, 10%, and 1,000%

(Measurement of viscosity)

Viscosity was measured using a T-bar spindle D at 25°C and 5 rpm.
Device: B8R type viscometer (helical stand used in combination, viscosity range 500 to 3,000 dPa, Toki Sangyo Co., Ltd.)

(Measurement of particle size of solid fat)

Examination was carried out using a microscope at 1,000x magnification with orthogonal polarized light, and the average of the size of 20 solid fat particles within the same field of view was defined as the average particle size (μm). The size of each solid fat particle was the average of the maximum diameter and the minimum diameter of the solid fat particle.

(Measurement of particle size of emulsified particles)

The diameter of 20 emulsified particles within the same field of view were directly measured in a digital image taken with a microscope under visible light, and the average thereof was defined as the emulsified particle size (μm).

(Evaluation of feel during use)

10 specialized cosmetic evaluation panelists carried out evaluation in terms of the presence/absence of 'richness', 'feel of spreading', and 'feel of thickness when spread', and the evaluation results were expressed as follows.
When 9 to 10 people had the feeling AA

When 7 to 8 people had the feeling BB
When 1 to 6 people had the feeling CC
When 0 people had the feeling DD

In Examples 1 to 5 and Comparative Examples 1 to 4, water-in-oil emulsified cosmetics having the formulations shown in Table 1 or 2(units wt%) were produced by the preparation methods below. Such water-in-oil emulsified cosmetics were subjected to the above-mentioned measurements and evaluations, and the results are shown in Table 3 or 4.

(Examples 1 to 3 and 5)

(Preparation method)

Component (B) was placed in a 2 L Agi Homo Mixer container and heated at 85°C. After confirming that component (B) had melted, components (A), (C), and (D) were added and stirring was carried out at 80 rpm. Subsequently, an aqueous phase containing component (E) heated to 85°C was added thereto, and emulsification was carried out using a homo-mixer at 8,000 rpm under reduced pressure conditions over 10 minutes. Following this, stirring was carried out at 100 rpm while maintaining the reduced pressure, and cooling to room temperature was carried out at a rate of cooling of 1.0°C/min.

(Example 4)

(Preparation method)

Emulsification was carried out in the same manner as in Example 1, and the rate of cooling after emulsification was set at 1.5°C/min.

(Comparative Example 1)

(Preparation method)

Production was carried out in the same manner as in Example 1 except that instead of component (C) of Example 1 silica having an average particle size of 4 μm was used, and the rotational speed of the homo-mixer was set at 5,000 rpm.

(Comparative Examples 2 to 4)

(Preparation method)

Production was carried out in the same manner as in Example 1 except that the rotational speed of the homo-mixer was set at 7,000 rpm, and the rate of cooling after emulsification was set at 1.5°C/min.

Subsequently, in Examples 6 to 9, water-in-oil emulsified cosmetics having the formulations shown in Table 5 (units wt%) were produced by the preparation methods below. Such water-in-oil emulsified cosmetics were subjected to the above-mentioned measurements and evaluations, and the results are shown in Table 6.

(Example 6)

(Preparation method)

Production was carried out in the same manner as in Example 1 except that the rotational speed of the homo-mixer was set at 10,000 rpm, and the rate of cooling after emulsification was set at 1.5°C/min.

(Example 7)

(Preparation method)

Production was carried out in the same manner as in Example 1 except that the rotational speed of the homo-mixer was set at 4,000 rpm, and the rate of cooling after emulsification was set at 0.5°C/min.

(Example 8)

(Preparation method)

Production was carried out in the same manner as in Example 1 except that the rotational speed of the homomixer was set at 7,000 rpm, and the rate of cooling after emulsification was set at 0.75°C/min.

(Example 9)

(Preparation method)

Production was carried out in the same manner as in Example 1 except that the rotational speed of the homomixer was set at 6,000 rpm, and the rate of cooling after emulsification was set at 1.0°C/min.

Subsequently, in Examples 10 to 12, water-in-oil emulsified cosmetics having the formulations shown in Table 7 (units wt%) were produced by the preparation method below. All of the water-in-oil emulsified cosmetics obtained in Examples 10 to 12 gave a feel of richness when applied to the skin. Such water-in-oil emulsified cosmetics were subjected to measurement of storage modulus, particle size of solid fat, and particle size of emulsified particles and evaluations in the same manner as described above. The results are shown in Table 8.

(Examples 10 to 12)

(Preparation method)

Production was carried out in the same manner as in Example 1 except that the rotational speed of the homomixer was set at 9,000 rpm, and the rate of cooling after emulsification was set at 1°C/min.

Table 1

| Component | Starting material | | Ex. 1 | Ex. 2 | Ex. 3 | Ex. 4 | Ex. 5 |
|---|---|---|---|---|---|---|---|
| A | Fluid isoparaffin | *A1 | 8 | 8 | 8 | 8 | 9 |
| A | Polydimethylsiloxane (viscosity 10 cs) | *A2 | 13 | 13 | 13 | 13 | 13 |
| A | 1-(2-Hydroxyethylamino)-3-isostearyloxy-2-propanol | *A3 | 0.2 | 0.2 | 0.2 | 0.2 | 0.2 |
| B | Cholesterol | *B1 | | | | | 1 |
| B | Palmitic acid | *B2 | 0.25 | 0.25 | 0.25 | 0.25 | 0.25 |
| B | Ceramide 2 | *B3 | | | | | |
| B | *N*-(Hexadecyloxyhydroxypropyl)-*N*-hydroxyethyl hexadecanamide | *B4 | 7 | 7 | 7 | 9 | 4 |
| C | HDI/trimethylolhexyllactone crosspolymer (plastic powder D-400) | *C1 | 2 | | | 0.5 | 0.6 |
| C | HDI/trimethylolhexyllactone crosspolymer (plastic powder D-800) | *C2 | | 2 | | | |
| C | Polymethylsilsesquioxane | *C3 | | | 2 | | |
| | Silica | *C8 | | | | | |
| D | Dimethicone copolyol (BY11-030) | *D1 | 5.2 | 5.2 | 5.2 | | 2.5 |

(continued)

| Component | Starting material | | Ex. 1 | Ex. 2 | Ex. 3 | Ex. 4 | Ex. 5 |
|---|---|---|---|---|---|---|---|
| D | Dimethicone copolyol (SH3775M) | *D2 | | | | 3 | 3 |
| E | Water | | Balance | Balance | Balance | Balance | Balance |
| | Glycerol | | 15 | 15 | 15 | 12 | 15 |
| | DPG | | 3 | 3 | 3 | 3 | 3 |
| | Total | | 100 | 100 | 100 | 100 | 100 |
| | (E)/((A) + (B) + (C)) | | 1.52 | 1.52 | 1.52 | 1.65 | 1.73 |
| | ((B) + (C))/(A) | | 0.44 | 0.44 | 0.44 | 0.46 | 0.26 |

Table 2

| Component | Starting material | | Comp. Ex. 1 | Comp. Ex. 2 | Comp. Ex. 3 | Comp. Ex. 4 |
|---|---|---|---|---|---|---|
| A | Fluid isoparaffin | *A1 | 18 | 8 | 8 | 8 |
| A | Polydimethylsiloxane (viscosity 10 cs) | *A2 | 13 | 13 | 13 | 13 |
| A | 1-(2-Hydroxyethylamino)-3-isostearyloxy-2-propanol | *A3 | 0.2 | 0.2 | 0.2 | |
| B | Cholesterol | *B1 | | | | |
| B | Palmitic acid | *B2 | 0.25 | 0.25 | 0.25 | |
| B | Ceramide 2 | *B3 | | | | |
| B | *N*-(Hexadecyloxyhydroxypropyl)-*N*-hydroxyethyl hexadecanamide | *B4 | 6 | 9 | 11 | |
| C | HDI/trimethylolhexyllactone crosspolymer (plastic powder D-400) | *CI | | | | |
| C | HDI/trimethylolhexyllactone crosspolymer (plastic powder D-800) | *C2 | | | 4.5 | 4.5 |
| C | Polymethylsilsesquioxane | *C3 | | | | |
| | Silica | *C8 | 1 | | | |
| D | Dimethicone copolyol (BY11-030) | *D1 | 5.2 | 5.2 | 5.2 | 5.2 |
| D | Dimethicone copolyol (SH3775M) | *D2 | | | | |
| E | Water | | Balance | Balance | Balance | Balance |
| | Glycerol | | 15 | 15 | 15 | 15 |
| | DPG | | 3 | 3 | 3 | 3 |
| | Total | | 100 | 100 | 100 | 100 |
| | (E)/((A) + (B) + (C)) | | 1.02 | 1.52 | 1.08 | 2.01 |
| | ((B) + (C))/(A) | | 0.20 | 0.44 | 0.74 | 0.21 |

Table 3

| | Ex. 1 | Ex. 2 | Ex. 3 | Ex. 4 | Ex. 5 |
|---|---|---|---|---|---|
| Average particle size of solid fat (B) (μm) | 7 | 7 | 7 | 9 | 5 |
| Emulsified particle size (μm) | 2 | 2 | 2 | 2.5 | 3 |
| Measurement value by B8R type viscometer (helical) (dPa·s) | 1450 | 1150 | 1250 | 1250 | 900 |
| Storage modulus G' when strain 0.1% | 1100 | 950 | 1050 | 1050 | 870 |
| Storage modulus G' when strain 10% | 1060 | 930 | 1020 | 1020 | 840 |
| Storage modulus G' when strain 1000 % | 40 | 30 | 36 | 60 | 30 |
| Storage modulus G' conditions 2 - conditions 3 | 1020 | 900 | 984 | 960 | 810 |
| Richness when applying | AA | BE | AA | AA | BB |
| Spreading when applying | AA | BE | BB | BB | BB |
| Feel of thickness when spread | AA | BE | BB | BB | BB |

Table 4

| | Comp. Ex. 1 | Comp. Ex. 2 | Comp. Ex. 3 | Comp. Ex. 4 |
|---|---|---|---|---|
| Average particle size of solid fat (B) (μm) | 6 | 10 | 13 | - |
| Emulsified particle size (μm) | 10 | 5 | 5 | 5 |
| Measurement value by B8R type viscometer (helical) (dPa·s) | 480 | 980 | 1800 | 1800 |
| Storage modulus G' when strain 0.1% | 385 | 790 | 1470 | 580 |
| Storage modulus G' when strain 10% | 380 | 730 | 1420 | 420 |
| Storage modulus G' when strain 1000% | 8 | 70 | 150 | 15 |
| Storage modulus G' conditions 2 - conditions 3 | 372 | 660 | 1270 | 405 |
| Richness when applying | DC | CC | BB | DC |
| Spreading when applying | CC | BB | DE | DD |
| Feel of thickness when spread | CC | CC | BB | CC |

Table 5

| Component | Starting material | | Ex. 6 | Ex. 7 | Ex. 8 | Ex. 9 |
|---|---|---|---|---|---|---|
| A | Fluid isoparaffin | *A1 | | 8 | 8 | |
| A | Polydimethylsiloxane (viscosity 10 cs) | *A2 | 10 | 10 | 8 | 10 |
| A | Squalane | *A4 | 10 | | | 10 |
| A | Neopentyl dicaprate glycol | *A5 | | | 0.2 | |
| A | Isostearyl isostearate | *A6 | | | 0.2 | |
| A | Olive oil | *A7 | | | 0.2 | |
| A | Uvinul MC80 | *A8 | | | 0.5 | |
| B | Cholesterol | *B1 | 0.5 | 0.5 | | 0.5 |
| B | Palmitic acid | *B2 | 0.5 | 0.5 | | |
| B | Ceramide 2 | *B3 | 0.5 | 0.5 | | |
| B | *N*-(Hexadecyloxyhydroxypropyl)-*N*-hydroxyethylhexadecanamide | *B4 | 9.5 | 7 | 7 | 6.5 |
| B | Paraffin wax 155 | *B5 | 0.4 | 0.4 | | |
| B | Stearic acid | *B6 | | | 0.5 | |
| B | Microcrystalline wax | *B7 | | | | 0.4 |
| B | Beeswax | *B8 | | | 0.1 | |
| B | Ceresine | *B9 | | | 0.1 | |
| B | Myristic acid | *B10 | | | | 0.5 |
| C | HDI/trimethylolhexyllactonecro sspolymer (plastic powder D-400) | *C1 | 2.5 | | | 2 |
| C | Polymethylsilsesquioxane | *C3 | | | | |
| C | Tospearl-3120 | *C4 | | 2 | 1 | |
| C | Nylon powder | *C5 | | | | 0.3 |
| C | Polymethyl methacrylate resin | *C6 | | | | 0.2 |
| C | Mica | *C7 | | | | 0.3 |
| D | Dimethicone copolyol (BY11-030) | *D1 | | | 6 | |
| D | Dimethicone copolyol (SH3775M) | *D2 | | 3 | | 4 |

(continued)

| Component | Starting material | | Ex. 6 | Ex. 7 | Ex. 8 | Ex. 9 |
|---|---|---|---|---|---|---|
| D | Dimethicone copolyol (BY25-337) | *D3 | 4 | | | |
| D | Dimethicone copolyol (FZ-2233) | *D4 | 1 | | | 2 |
| E | Water | | Balance | Balance | Balance | Balance |
| | Glycerol | | 10 | 10 | 10 | 10 |
| | DPG | | 5 | 5 | 5 | 5 |
| | Total | | 100 | 100 | 100 | 100 |
| | (E)/((A) + (B) + (C)) | | 1.36 | 1.84 | 2.06 | 1.57 |
| | ((B) + (C)) / (A) | | 0.70 | 0.61 | 0.51 | 0.54 |

Table 6

| Component | Starting material | Ex. 6 | Ex. 7 | Ex. 8 | Ex. 9 |
|---|---|---|---|---|---|
| | Average particle size of solid fat (B) (μm) | 5 | 13 | 8 | 7 |
| | Emulsified particle size (μm) | 0.83 | 12.5 | 4 | 2 |
| | Measurement value by B8R type viscometer (helical) (dPa·s) | 2300 | 1200 | 950 | 1800 |
| | Storage modulus G' when strain 0.1% | 1950 | 900 | 880 | 1600 |
| | Storage modulus G' when strain 10% | 1900 | 860 | 840 | 1550 |
| | Storage modulus G' when strain 1000% | 220 | 40 | 20 | 120 |
| | Storage modulus G' conditions 2 - conditions 3 | 1680 | 820 | 820 | 1430 |
| | Richness when applying | AA | AA | BB | BE |

Table 7

| Component | Starting material | | Ex. 10 | Ex. 11 | Ex. 12 |
|---|---|---|---|---|---|
| A | Fluid isoparaffin | *A1 | 20 | | |
| | Polydimethylsiloxane (viscosity 10 cs) | *A2 | 20 | 6 | 10 |
| | Squalane | *A4 | | 5.5 | 5 |
| B | Ceramide 2 | *B3 | | | 5 |
| | *N*-(Hexadecyloxyhydroxyprop yl)-*N*-hydroxyethylhexadecanami de | *B4 | 12 | 3 | |
| | Myristic acid | *B1 0 | 1 | | |
| C | HDI/trimethylolhexyllact one crosspolymer (plastic powder D-400) | *C1 | 6.5 | | |
| | HDI/trimethylolhexyllact one crosspolymer (plastic powder D-800) | *C2 | | 2.8 | |
| | Tospearl-3120 | *C4 | | | 4.9 |
| D | Dimethicone copolyol (BY11-030) | *D1 | 8 | | |
| | Dimethicone copolyol (SH3775M) | *D2 | | 8 | |
| | PEG5 hydrogenated castor oil (Emalex HC-5) | *D5 | | 2 | |
| | Dimethicone copolyol (FZ-2233) | *D4 | | | 3 |
| E | Water balance | | Balance | Balance | Balance |
| | Glycerol | | 10 | 23.5 | 15 |
| | DPG | | | 10 | 3 |
| | Total | | 100 | 100 | 100 |
| | E: (A + B + C) | | 0.38 | 2.27 | 2.17 |
| | (B + C)/A | | 0.49 | 0.50 | 0.66 |

Table 8

| Component | Starting material | Ex. 10 | Ex. 11 | Ex. 12 |
|---|---|---|---|---|
| | Average particle size of powder (C) (μm) | 13 | 8 | 15 |
| | Average particle size of solid fat (B) (μm) | 12 | 1 | 14 |
| | Emulsified particle size (μm) | 0.15 | 0.3 | 13 |
| | Measurement value by B8R type viscometer (helical) (dPa·s) | 2850 | 870 | 980 |
| | Richness when applying | AA | BB | AA |
| | Spreading when applying | BB | BB | BB |
| | Feel of thickness when spread | AA | BB | AA |

*A1 ParLeam EX, The Nisshin OilliO Group, Ltd.
*A2 Polydimethylsiloxane (viscosity 10 cs) (Shin-Etsu Chemical Co., Ltd.)
*A3 1-(2-Hydroxyethylamino)-3-isostearyloxy-2-propanol, Kao Corporation
*A4 Squalane, Nikko Chemicals Co., Ltd.
*A5 Estemol N-01 The Nisshin OilliO Group, Ltd.
*A6 ISIS Kokyu Alcohol Kogyo Co., Ltd.
*A7 Cropure OL Croda Japan
*A8 Octyl methoxycinnamate BASF Japan
*B1 Cholesterol, Nippon Fine Chemical, melting point 148°C
*B2 Palmitic acid, Kao Corporation, melting point 60°C to 63°C
*B3 Croda Japan, melting point 100°C to 105°C
*B4 Kao Corporation, melting point 70°C to 75°C
*B5 Nippon Seiro Co., Ltd, melting point 68.3°C to 70.9°C
*B6 Stearic acid, Kao Corporation, melting point 67°C to 69°C
*B7 Multiwax W-445 SONNEBORN Inc., melting point 60°C to 85°C
*B8 BEESWAX Croda Japan, melting point 60°C to 67°C
*B9 Ceresine wax SP-1020P STRAHL & PITSCH Inc., melting point 61°C to 95°C
*B10 Myristic acid, Kao Corporation, melting point 52°C to 54°C
*C1 Hexamethylene diisocyanate/trimethylolhexyllactone crosslinked polymer and anhydrous silicic acid mixture, average particle size 13 μm
*C2 Hexamethylene diisocyanate/trimethylolhexyllactone crosslinked polymer and anhydrous silicic acid mixture, average particle size 8 μm
*C3 Polymethylsilsesquioxane, Momentive Performance Materials Japan LLC, average particle size 15 μm
*C4 Polymethylsilsesquioxane, Momentive Performance Materials Japan LLC, average particle size 12 μm
*C5 Nylon HK-5000 Sumika Enviro-Science Co., Ltd., average particle size 8 μm
*C6 Matsumoto Microsphere M-100 Matsumoto Yushi Seiyaku Co., Ltd., 10.5 μm
*C7 SA-310 Yamaguchi Mica Co., Ltd. average particle size 26 μm
*C8 Sunsphere NP-30 Miyoshi Kasei Inc. average particle size 4 μm
*D1 Dow Corning Toray Co., Ltd., HLB 3
*D2 Dow Corning Toray Co., Ltd., HLB 5
*D3 Dow Corning Toray Co., Ltd., HLB 2
*D4 Dow Corning Toray Co., Ltd., HLB 2.5
*D5 Nihon Emulsion Co., Ltd., HLB 5

## Claims

**1.** A water-in-oil emulsified cosmetic comprising at least components (A) to (E) below:

(A) an oil that is a liquid at 25°C, which comprises a polydimethylsiloxane that is of a viscosity no greater than 50 cSt at 25°C, or fluid isoparaffin,
(B) an oil that is a solid at 25°C, which comprises a ceramide or a ceramide-like compound represented by

formula (1),
(C) a hydrophobic powder having an average particle size of 8 to 30 μm, which comprises a urethane resin or silicone resin,
(D) a surfactant having an HLB of no greater than 5, which comprises a polyether-modified silicone; and
(E) water,

component (A) having a content of 10 to 50 wt%,
component (B) having a content of 0.5 to 15 wt%,
component (C) having a content of 0.5 to 10 wt%, and
component (D) having a content of 0.2 to 10 wt%, the proportions by weight ratio of components (A) to (E) satisfying the relational expressions (1) to (3) below,

$$(E):((A)+(B)+(C)) = 10:90 \text{ to } 70:30 \qquad (1)$$

$$0.25 \leq ((B)+(C))/(A) \leq 0.7 \qquad (2)$$

$$(B) > (C) \qquad (3),$$

emulsified particles, emulsified from an aqueous phase component comprising component (E), having an average emulsified particle size of 0.1 to 25 μm,
the relationship in size of said average emulsified particle size, average particle size of component (B), and average particle size of component (C) being (average particle size of component (C)) > (average particle size of component (B)) > (said average emulsified particle size), and
the cosmetic having a viscosity measured at 25°C and 5 rpm using a T-bar spindle D of 500 to 3,000 dPa·s;
wherein formula (1) is:

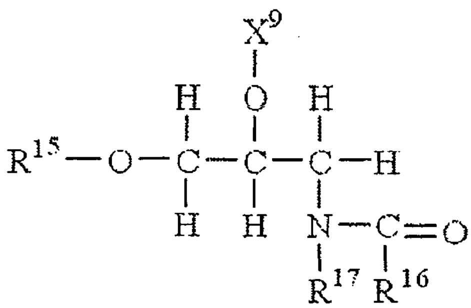

(1)

in the formula, $R^{15}$ denotes a hydrogen atom or a straight-chain, branched-chain, or cyclic saturated or unsaturated hydrocarbon group having 10 to 22 carbon atoms that may optionally be substituted with a hydroxy group;
$X^9$ denotes a hydrogen atom, an acetyl group, or a glyceryl group;
$R^{16}$ denotes a straight-chain, branched-chain, or cyclic saturated or unsaturated hydrocarbon group having 5 to 22 carbon atoms that may optionally be substituted with a hydroxy group or an amino group, or one in which a straight-chain or branched-chain saturated or unsaturated fatty acid having 8 to 22 carbon atoms that may optionally be substituted with a hydroxy group is ester-bonded to the ω terminal of the above hydrocarbon group; and
$R^{17}$ denotes a hydrogen atom or an alkyl group having a total of 1 to 30 carbon atoms that may optionally be substituted with a hydroxy group, a hydroxyalkoxy group, an alkoxy group, or an acetoxy group.

**2.** The cosmetic as set forth in Claim 1, wherein component (A) comprises fluid isoparaffin and silicone oil.

**3.** The cosmetic as set forth in Claim 1 or 2, wherein the relational expression (1) is 27:73 to 70:30.

## Patentansprüche

1. Emulgiertes Wasser-in-Öl-Kosmetikum, enthaltend zumindest die Komponenten (A) bis (E) unten:

(A) ein Öl, das bei 25°C flüssig ist, das ein Polydimethylsiloxan enthält, das eine Viskosität bei 25°C von nicht mehr als 50 cSt enthält, oder ein flüssiges Isoparaffin,
(B) ein Öl, das ein Feststoff bei 25°C ist, das ein Ceramid oder eine Ceramid-artige Verbindung mit der Formel (1) enthält,
(C) ein hydrophobes Pulver mit einer durchschnittlichen Teilchengröße von 8 bis 30 μm, das ein Urethanharz oder Silikonharz enthält,
(D) ein Tensid mit einem HLB von nicht mehr als 5, das ein Polyether-modifiziertes Silikon enthält, und
(E) Wasser,
wobei die Komponente (A) einen Gehalt von 10 bis 50 Gew.-%, die Komponente (B) einen Gehalt von 0,5 bis 15 Gew.-%, die Komponente (C) einen Gehalt von 0,5 bis 10 Gew.-% und die Komponente (D) einen Gehalt von 0,2 bis 10 Gew.-% hat,
wobei die Gewichtsverhältnisse der Anteile der Komponenten (A) bis (E) die Ausdrücke (1) bis (3) unten erfüllen:

$$(E):(A) + (B) + (C)) = 10:90 \text{ bis } 70:30 \qquad (1)$$

$$0,25 \leq ((B) + (C))/(A) \leq 0,7 \qquad (2)$$

$$(B) > (C) \qquad (3)$$

wobei emulgierte Teilchen, emulgiert von einer wässrigen Phasenkomponente, die die Komponente (E) enthält, eine durchschnittliche emulgierte Teilchengröße von 0,1 bis 25 μm aufweisen,
die Beziehung der Größe der durchschnittlichen emulgierten Teilchengröße, der durchschnittlichen Teilchengröße der Komponente (B) und der durchschnittlichen Teilchengröße der Komponente (C) (durchschnittliche Teilchengröße der Komponente (C)) > (durchschnittliche Teilchengröße der Komponente (B)) > (durchschnittliche emulgierte Teilchengröße) erfüllt, und
worin das Kosmetikum eine Viskosität, gemessen bei 25°C und 5 UpM unter Verwendung einer T-Stangenspindel C von 500 bis 3.000 dPa·s aufweist, worin die Formel (1) ist:

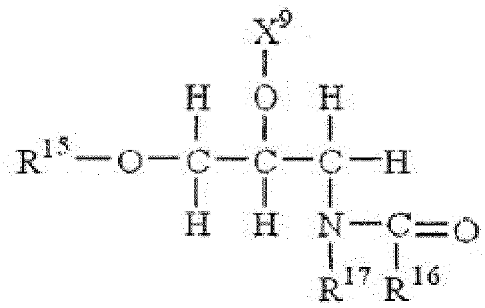

(1)

worin in der Formel $R^{15}$ ein Wasserstoffatom oder eine geradkettige, verzweigte oder cyclische, gesättigte oder ungesättigte Kohlenwasserstoffgruppe mit 10 bis 22 Kohlenstoffatomen ist, die wahlweise mit einer Hydroxygruppe substituiert sein kann,
$X^9$ ein Wasserstoffatom, Acetylgruppe oder Glycerylgruppe ist,
$R^{16}$ eine geradkettige, verzweigte oder cyclische, gesättigte oder ungesättigte Kohlenwasserstoffgruppe mit 5 bis 22 Kohlenstoffatomen ist, die wahlweise mit einer Hydroxygruppe oder einer Aminogruppe substituiert sein kann, oder eine ist, worin eine geradkettige, verzweigte, gesättigte oder ungesättigte Fettsäure mit 8 bis 22 Kohlenstoffatomen, die wahlweise substituiert sein kann mit einer Hydroxygruppe, an das ω-Ende der obigen Kohlenwasserstoffgruppe Ester-gebunden ist; und
$R^{17}$ ein Wasserstoffatom oder eine Alkylgruppe mit insgesamt 1 bis 30 Kohlenstoffatomen ist, die wahlweise mit einer Hydroxygruppe, Hydroxyalkoxygruppe, Alkoxygruppe oder Acetoxygruppe substituiert sein kann.

**2.** Kosmetikum gemäß Anspruch 1, worin die Komponente (A) flüssiges Isoparaffin und Silikonöl enthält.

**3.** Kosmetikum gemäß Anspruch 1 oder 2, worin der Ausdruck (1) 27:73 bis 70:30 ist.

**Revendications**

**1.** Cosmétique émulsifié eau dans huile comprenant au moins les composants (A) à (E) ci-dessous :

(A) une huile qui est un liquide à 25°C, qui comprend un polydiméthylsiloxane qui est d'une viscosité non supérieure à 50 cSt à 25°C, ou de l'isoparaffine fluide,
(B) une huile qui est un solide à 25°C, qui comprend un céramide ou un composé de type céramide représenté par la formule (1),
(C) une poudre hydrophobique ayant une taille de particule moyenne de 8 à 30 μm, qui comprend une résine uréthane ou un résine silicone,
(D) un tensioactif ayant un équilibre hydrophile-lipophile (HLB) on supérieur à 5, qui comprend une silicone modifiée par un polyéther ;
(E) de l'eau,

le composant (A) ayant une teneur de 10 à 50% en poids,
le composant (B) ayant une teneur de 0,5 à 15% en poids,
le composant (C) ayant une teneur de 0,5 à 10% en poids,
et
le composant (D) ayant une teneur de 0,2 à 10% en poids,

les proportions en rapport en poids des composants (A) à (E) satisfaisant les expressions relationnelles (1) à (3) ci-dessous,

$$(E):((A) + (B) + (C)) = 10:90 \text{ à } 70:30 \qquad (1)$$

$$0{,}25 \leq ((B) + (C))/(A) \leq 0{,}7 \qquad (2)$$

$$(B) > (C)$$

des particules émulsifiées, émulsifiées à partir d'un composant de phase aqueuse comprenant le composant (E), ayant une taille de particule émulsifiée moyenne de 0,1 à 25 μm,
la relation en taille de ladite taille de particule émulsifiée moyenne, de la taille de particule moyenne du composant (B), et de la taille de particule moyenne du composant (C) étant
(taille de particule moyenne du composant (C)) > (taille de particule moyenne du composant (B)) > (dite taille de particule émulsifiée moyenne), et
le cosmétique ayant une viscosité mesurée à 25°C et 5 tours par minute (rpm) en utilisant une broche D en T de 500 à 3000 dPa·s ;
dans lequel la formule (1) est :

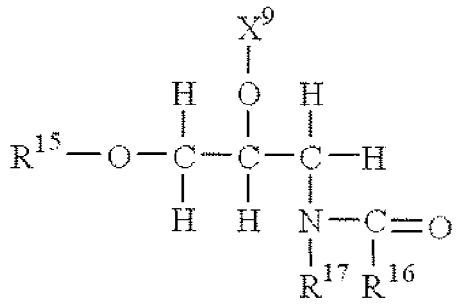

(1)

dans la formule, $R^{15}$ dénote un atome d'hydrogène ou une chaîne linéaire, une chaîne ramifiée, ou un groupe hydrocarboné saturé ou insaturé cyclique ayant de 10 à 22 atomes de carbone qui peut optionnellement être substitué avec un groupe hydroxyle ;
$X^9$ dénote un atome d'hydrogène, un groupe acétyle, ou un groupe glycéryle ;
$R^{16}$ dénote une chaîne linéaire, une chaîne ramifiée, ou un groupe hydrocarboné saturé ou insaturé cyclique ayant de 5 à 22 atomes de carbone qui peut optionnellement être substitué avec un groupe hydroxyle ou un groupe amino ; ou l'un dans lequel un acide gras saturé ou insaturé à chaîne linéaire ou à chaîne ramifiée ayant de 8 à 22 atomes de carbone qui peut optionnellement être substitué avec un groupe hydroxyle qui est lié comme par un ester au terminus ω du groupe hydrocarboné ci-dessus ; et
$R^{17}$ dénote un atome d'hydrogène ou un groupe alkyle ayant un total de 1 à 30 atomes de carbone qui peut optionnellement être substitué avec un groupe hydroxyle, un groupe hydroxyalkoxy, un groupe alkoxy, ou un groupe acétoxy.

**2.** Le cosmétique tel que défini dans la Revendication 1, dans lequel le composant (A) comprend de l'isoparaffine fluide ou de l'huile de silicone.

**3.** Le cosmétique tel que défini dans la Revendication 1 ou 2, dans lequel l'expression relationnelle (1) est de 27:73 à 70:30.

FIG. 1

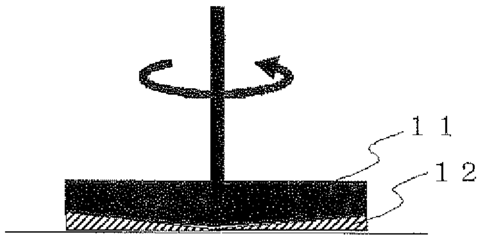

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7267819 A
- JP 2006282632 A
- JP 5262622 A
- JP 8104613 A
- WO 2007064687 A